# EUROPEAN PATENT APPLICATION

(11) **EP 4 715 006 A2**
(43) Date of publication of application: **25.03.2026**
(21) Application number: 25200422.1
(22) Date of filing: 05.09.2025
(51) Int. Cl.: C08K 5/14, C08K 5/07, C08K 5/08, C08K 5/09, C08K 5/10, C08L 23/0853, C08L 23/06, C08L 23/08

(54) **UNSATURATED SCORCH RETARDERS**

(30) Priority: 19.09.2024 EP 24201390
(71) Applicant: Nouryon Chemicals International B.V., 1101 BZ Amsterdam (NL)
(72) Inventor: BEEK, Waldo Joseph Elisabeth, 1101 BZ Amsterdam (NL); TEMPEL, Kasper Jakob, 1101 BZ Amsterdam (NL)
(74) Representative: Ingrassia, Fisher & Lorenz UK Ltd.

(57) **Abstract**

The present disclosure relates to organic peroxide formulation comprising at least one organic peroxide; and at least one unsaturated scorch retarder having the structure of general formula (I): 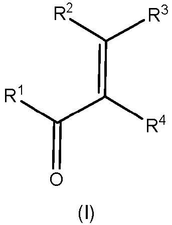 wherein, R¹ is selected from OH, O-R⁵, or a saturated or unsaturated, linear, branched, or cyclic C₁ to C₈ alkyl group; R² is on each occurrence independently selected from H or a saturated or unsaturated, linear or branched C₁ to C₈ alkyl group; R³ is on each occurrence independently selected from H, O-R⁶, or a saturated or unsaturated, linear or branched C₁ to C₈ alkyl group; R⁴ is on each occurrence independently selected from OH, O-R⁶, or a saturated or unsaturated, linear or branched C₁ to C₈ alkyl group; R⁵ is selected from a saturated or unsaturated, linear or branched C₁ to C₈ alkyl group or -[CH₂]ₙ-OCOCR⁴CR²R³; R⁶ is on each occurrence independently selected from a saturated or unsaturated, linear or branched C₁ to C₈ alkyl group; and n is from 2-8, with the proviso that one but not both of R² and R³ are H, or wherein R¹ and R² are joined to form a 5, 6, or 7 membered ring, said ring being formed of atoms selected from C, O, and S and containing one or two carbon-carbon double bonds and one or two carbonyl groups, said ring being optionally substituted with one or more saturated or unsaturated, linear or branched C₁ to C₈ alkyl groups; R³ is H; and R⁴ and R⁶ are as described above, or wherein R³ and R⁴ are joined to form a 5, 6, or 7 membered ring, said ring being formed of atoms selected from C, O, and S and containing one or two carbon-carbon double bonds, said ring optionally substituted with one or more saturated or unsaturated, linear or branched C₁ to C₈ alkyl groups; R² is H; and R¹, R⁵, R⁶ and n are as described above.

## Description

### Technical field

The present invention relates to organic peroxide formulations and to polymer compositions with increased scorch protection.

### Background

"Scorch" refers to the premature or unwanted crosslinking or modification of a peroxide-containing polymer composition that occurs during processing of the composition at a temperature above room temperature but below the final cure temperature. When scorch occurs, problems result including poor dispersion of ingredients, increased viscosity, incomplete mold filling, and the creation of defective parts and scrap.

"Scorch time" is the safe processing time window at a particular temperature before the onset of this premature or unwanted crosslinking (scorch). For many applications, it is critical to know the scorch time of a peroxide-containing polymer composition at a temperature approximately 40 °C lower than the final cure temperature (typically referred to as the "safe processing temperature").

Scorch time can be determined or estimated using several methods, for example, with a rheometer. It is also common to use the so-called "Mooney scorch test" (ISO 289-2:1994 (Mooney scorch)). In this test, an amount of peroxide-containing polymer composition is placed in a heated mold (typically at the "safe processing temperature") with a rotor fully embedded in the polymer composition. As the rotor is rotated, the resistance to its rotation (torque) is measured in Mooney Units (MU). Premature crosslinking leads to an increase in viscosity and a higher MU readout in time. The "Mooney Scorch" standard describes two relevant parameters, "T5" and "T35", referring to the time to reach 5 or 35 MU higher than the minimum Mooney viscosity, respectively (the curve first drops through a minimum as a result of heating the sample, and then slowly starts to increase to T5 and T35). The longer those times, the wider the safe processing time window.

There have been several attempts to extend scorch time. For example, by using a scorch retarder alongside the peroxide. WO 2002/28946 A1, for instance, discloses a scorch retarding composition comprising a nitroxide and at least one promoter compound containing a double bond capable of being bifunctional or polyfunctional. In its examples, a mixture of low-density polyethylene (LDPE), dicumyl peroxide (DCP), 2,2,6,6 tetramethylpiperidinyloxy (TEMPO) and diisopropenylbenzene (DIPB), is shown to have extended scorch time.

More recently, WO 2021/207525 A1 has suggested the use of alternative scorch retarders that are derived or derivable from natural sources, in particular, for use in products that comply with various governmental indirect food contact, FDA skin contact or medical applications, or National Sanitation Foundation guidelines. However, such scorch retarders have primarily been optimized for EPDM rubber applications.

There remains a need for scorch protected peroxide formulations that are useful in other applications, such as with polymers commonly used in solar, shoe-soling, or wire and cable applications.

### Description

We have now found a group of unsaturated compounds that work surprisingly well as scorch retarders in peroxide-based polymer crosslink systems that typically find use in solar, shoe-soling, or wire and cable applications, without having a detrimental (or unacceptable) effect on the final cure.

Accordingly, in a first aspect, the present disclosure is directed to an organic peroxide formulation comprising, consisting of, or consisting essentially of:
at least one organic peroxide; and
at least one unsaturated scorch retarder having the structure of general formula (I): 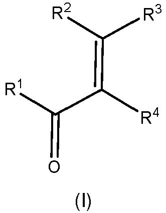 wherein,
   R¹ is selected from OH, O-R⁵, or a saturated or unsaturated, linear, branched, or cyclic C₁ to C₈ alkyl group;
   R² is on each occurrence independently selected from H or a saturated or unsaturated, linear or branched C₁ to C₈ alkyl group;
   R³ is on each occurrence independently selected from H, O-R⁶, or a saturated or unsaturated, linear or branched C₁ to C₈ alkyl group;
   R⁴ is on each occurrence independently selected from OH, O-R⁶, or a saturated or unsaturated, linear or branched C₁ to C₈ alkyl group;
   R⁵ is selected from a saturated or unsaturated, linear or branched C₁ to C₈ alkyl group or -[CH₂]ₙ-OCOCR⁴CR²R³;
   R⁶ is on each occurrence independently selected from a saturated or unsaturated, linear or branched C₁ to C₈ alkyl group; and
   n is from 2-8, preferably from 3-6, and most preferably 6,
   with the proviso that one but not both of R² and R³ are H,
      or wherein
   R¹ and R² are joined to form a 5, 6, or 7 membered ring, said ring being formed of atoms selected from C, O, and S and containing one or two carbon-carbon double bonds and one or two carbonyl groups, said ring being optionally substituted with one or more saturated or unsaturated, linear or branched C₁ to C₈ alkyl group;
   R³ is H; and
   R⁴ and R⁶ are as described above,
      or wherein
   R³ and R⁴ are joined to form a 5, 6, or 7 membered ring, said ring being formed of atoms selected from C, O, and S and containing one or two carbon-carbon double bonds, optionally substituted with one or more saturated or unsaturated, linear or branched C₁ to C₈ alkyl group;
   R² is H; and
   R¹, R⁵, R⁶ and n are as described above.

Advantageously, the unsaturated scorch retarders of the first aspect of the invention have been found to extend the safe processing window of many organic peroxides, including those commonly used to crosslink polymers with solar, shoe-soling, or wire and cable applications. Without wishing to be bound by theory, it is believed that by having only one hydrogen in the beta position to the carbonyl group, in combination with substituent R⁴ in the alpha position, unsaturated scorch retarders having the structure of general formula (I) can react readily with peroxide free radicals, or with polymer radicals after reaction of the peroxide with the polymer, to form free radicals on the scorch retarder, which are stabilized with respect to further reaction (e.g., with a polymer).

As used herein, the expression "saturated or unsaturated, linear or branched C₁ to C₈ alkyl group" at least includes substituents selected from methyl, ethyl, n-propyl, isopropyl, n-butyl, sec-butyl, isobutyl, tert-butyl, n-pentyl, isopentyl, neopentyl, tert-pentyl, 1-methylbutyl, 1-ethylpropyl, 1,2-dimethylpropyl, 2-methylbutyl, n-hexyl, isohexyl, neohexyl, tert-hexyl, 1-methylpentyl, 1-ethylbutyl, 1-ethyl-2-methylpropyl, 1,3-dimethylbutyl, n-heptyl, n-octyl, ethenyl, prop-1-enyl, prop-2-enyl, prop-1-en-2-yl, but-1-enyl, but-2-enyl, but-3-enyl, but-1-en-2-yl, but-2-en-2-yl, but-3-en-2-yl, pent-1-enyl, pent-2-enyl, pent-3-enyl, pent-4-enyl, hex-1-enyl, hex-2-enyl, hex-3-enyl, hex-4-enyl, hex-5-enyl, heptenyl and octenyl. Likewise, the expression "a saturated or unsaturated cyclic C₁ to C₈ alkyl group" at least includes substituents selected from cyclopropyl, cyclobutyl, cyclopentyl, cyclopentenyl, cyclohexyl, cyclohexenyl, cycloheptyl, and cyclooctyl.

In preferred embodiments, the at least one unsaturated scorch retarder has the structure of general formula (I), wherein:
R¹ is selected from OH, O-R⁵, or a saturated, linear C₁ to C₄ alkyl group;
R² is on each occurrence independently selected from H or a saturated, linear C₁ to C₄ alkyl group;
R³ is on each occurrence independently selected from H, O-R⁶, or a saturated C₁ to C₃ alkyl group;
R⁴ is on each occurrence independently selected from OH, O-R⁶, or a saturated, linear C₁ to C₄ alkyl group;
R⁵ is selected from a saturated, linear C₁ to C₄ alkyl group or -[CH₂]ₙ-OCOCR⁴CR²R³;
R⁶ is on each occurrence independently a saturated, linear C₁ to C₄ alkyl group; and
n is from 3-6, preferably 6,
with the proviso that one but not both of R² and R³ are H.

As used herein, the expression "saturated, linear C₁ to C₄ alkyl group" includes substituents selected from methyl, ethyl, n-propyl and n-butyl, while the expression "saturated C₁ to C₃ alkyl group" includes substituents selected from methyl, ethyl, and n-propyl.

More preferably, the at least one unsaturated scorch retarder has the structure of general formula (I), wherein R¹ is selected from OH or O-R⁵; R² is H; R³ and R⁴ are on each occurrence independently selected from methyl or ethyl, preferably methyl; R⁵ is selected from methyl, ethyl, n-propyl and n-butyl or -[CH₂]ₙ-OCOCR⁴CR²R³; and n is 6. Such compounds, for example, ethyl *trans*-2-methyl-2-butenoate, *trans*-2-methyl-2-butenoic acid, and hexane-1,6-diyl (2E,2'E)-bis(2-methylbut-2-enoate, have been found to perform well as scorch retarders, reacting readily with peroxide free radicals, or with polymer radicals after reaction of the peroxide with the polymer, and forming stable free radicals on the scorch retarder.

Alternatively, the at least one unsaturated scorch retarder preferably has the structure of general formula (I), wherein:
R³ and R⁴ are joined to form a 6 membered ring, said ring being formed of atoms selected from C, O, and S and containing one or two carbon-carbon double bonds, optionally substituted with one or more saturated, linear C₁ to C₄ alkyl group;
R¹ is selected from OH, O-R⁵, or a saturated, linear C₁ to C₄ alkyl group;
R² is H;
R⁵ is selected from a saturated, linear C₁ to C₄ alkyl group or -[CH₂]ₙ-OCOCR⁴CR²R³;
R⁶ is on each occurrence independently a saturated, linear C₁ to C₄ alkyl group; and
n is from 3-6, preferably 6.

When R³ and R⁴ are joined to form a 6 membered ring, the ring is preferably formed of C atoms and contains a single carbon-carbon double bond. In addition, R¹ is preferably OH, methyl or ethyl. A preferred compound with this structure is 1-acetyl cyclohexene.

Alternatively, the at least one unsaturated scorch retarder preferably has the structure of general formula (I), wherein:
R¹ and R² are joined to form a 5 or 6 membered ring, said ring being formed of atoms selected from C, O, and S and containing one or two carbon-carbon double bonds and one or two carbonyl groups, said ring being optionally substituted with one or more saturated or unsaturated, linear or branched C₁ to C₄ alkyl group;
R³ is H;
R⁴ is selected from OH, O-R⁶, or a saturated, linear C₁ to C₄ alkyl group; and
R⁶ is on each occurrence independently a saturated, linear C₁ to C₄ alkyl group.

When R¹ and R² are joined to form a 6 membered ring, the at least one unsaturated scorch retarder preferably has the structure of general formula (IA) or (IB): 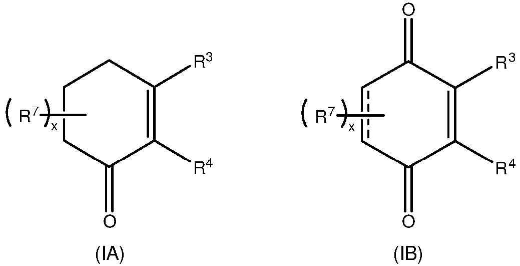 wherein
R³ is H;
R⁴ is selected from OH, O-R⁶, or a saturated, linear C₁ to C₄ alkyl group;
R⁶ is on each occurrence independently a saturated, linear C₁ to C₄ alkyl group;
R⁷ is on each occurrence independently selected from OH, O-R⁶ or a saturated or unsaturated, linear or branched C₁ to C₈ alkyl group;
x is from 0-4, preferably 0-3, more preferably 1-3; and
the dotted line in formula (IB) is an optional double bond.

More preferably, the at least one unsaturated scorch retarder has the structure of general formula (IA) or (IB), wherein R³ is H; R⁴ is selected from methyl or ethyl, preferably methyl; R⁷ is selected from methyl, ethyl, n-propyl, isopropyl, ethenyl, prop-1-enyl, prop-2-enyl, prop-1-en-2-yl; and x is 1, 2 or 3. Such compounds, for example, L-carvone, D-carvone, 2,6-dimethylbenzoquinone, 2,5-dimethylbenzoquinone, and ketoisophorone, have been found to perform well as scorch retarders, reacting readily with peroxide free radicals and forming stable free radicals on the scorch retarder.

When R¹ and R² are joined to form a 5 membered ring, the ring is preferably formed of atoms selected from C and O and contains one carbon-carbon double bond and one carbonyl group. In addition, R⁴ is preferably OH, methyl or ethyl. Preferred compounds with this structure are 3-Methyl-2(*5H*)-furanone and 2-Methyl-2-cyclopenten-1-one.

The organic peroxide formulations according to the first aspect of the invention typically contain from 30 to 95 wt.% of organic peroxide and from 5 to 30 wt.% of unsaturated scorch retarder, based on the total weight of the formulation. Preferably, the amount of organic peroxide in the formulation is from 30 to 92 wt.% and the amount of unsaturated scorch retarder is from 10 to 25 wt.%.

The organic peroxide formulations according to the first aspect of the invention preferably contain at least one organic peroxide selected from peroxyesters, percarbonates, peroxyketals, and dialkyl peroxides. The peroxides may be liquid or solid.

Examples of suitable dialkyl peroxides include tertiary alkyl organic peroxides, such as 2,5-dimethyl-2,5-di(tert-butylperoxy) hexane, 2,5-dimethyl-2,5-di(tert-butylperoxy) hexyne-3, and di-tert-butyl peroxide, tertiary aralkyl organic peroxides, such as dicumyl peroxide and di(tert-butylperoxyisopropyl) benzene, and tertiary alkyl/aralkyl organic peroxides, such as tert-butyl cumyl peroxide.

Examples of suitable percarbonates include peroxymonocarbonates, such as tert-butylperoxy 2-ethylhexyl carbonate, tert-amylperoxy 2-ethylhexyl carbonate, and tert-butyl peroxy isopropyl carbonate, and peroxy dicarbonates, such as dicetyl peroxydicarbonate and di(4-tert-butylcyclohexyl) peroxydicarbonate.

Examples of suitable peroxyketals (perketals) include 1,1-di(tert-butylperoxy)-3,3,5-trimethylcyclohexane, butyl 4,4-di(tert-butylperoxy) valerate and 1,1-di(tert-butylperoxy) cyclohexane.

Examples of suitable peroxyesters include tert-butyl peroxybenzoate, tert-butyl peroxy-2-ethylhexanoate, t-butyl peroxyacetate, tert-amyl peroxy-2-ethylhexanoate, 1,1,3,3-tetramethylbutyl peroxy-2-ethylhexanoate, tert-butyl peroxydiethylacetate and tert-butyl peroxyisobutyrate.

The organic peroxide formulations according to the first aspect of the invention preferably contain an organic peroxide selected from tert-butylperoxy 2-ethylhexyl carbonate, tert-amylperoxy 2-ethylhexyl carbonate, tert-butyl peroxy isopropyl carbonate, 1,1-di(tert-butylperoxy)-3,3,5-trimethylcyclohexane, di-tert-butyl peroxide, 1,1-di(tert-butylperoxy)cyclohexane, butyl 4,4-di(tert-butylperoxy)valerate, tert-butyl peroxybenzoate, tert-butyl cumyl peroxide, 2,5-dimethyl-2,5-di(tert-butylperoxy)hexane, 2-5-dimethyl-2,5-di(tert-butylperoxy)hexyne-3, t-butyl peroxyacetate, tert-butyl peroxy-2-ethylhexanoate, tert-amyl peroxy-2-ethylhexanoate, tert-butyl peroxydiethylacetate, tert-butyl peroxyisobutyrate, 1,1,3,3-tetramethylbutyl peroxy-2-ethylhexanoate,and mixtures thereof.

Preferably, the organic peroxide formulations according to the first aspect of the invention include an organic peroxide selected from tert-butyl cumyl peroxide, tert-butylperoxy 2-ethylhexyl carbonate, tert-amylperoxy 2-ethylhexyl carbonate 1,1-di(tert-butylperoxy)-3,3,5-trimethylcyclohexane, 1,1-di(tert-butylperoxy) cyclohexane, and mixtures thereof.

The organic peroxide formulations of the first aspect of the invention can be produced in a conventional manner. Typically, the ingredients of the formulation are mixed using well-known blending equipment, such as kettles or tanks with impellors, agitators, or in-tank low- or high-shear mixers, static inline mixers, and high-speed mixers. The kettles, tanks or mixers may be heated to aid the mixing. Because the peroxides may not be stable at higher temperatures, the mixing should be done well below the decomposition temperature of the organic peroxide.

The organic peroxide formulations may contain only organic peroxide and unsaturated scorch retarder. Such formulations can be prepared, for example, by mixing the unsaturated scorch retarder into the organic peroxide. Preferably, these organic peroxide formulations contain from 70 to 95 wt.% of organic peroxide and from 5 to 30 wt.% of unsaturated scorch retarder, based on the total weight of the formulation.

Alternatively, the organic peroxide formulations according to the first aspect of the invention optionally may contain other additives, including phlegmatisers, antiozonants, antioxidants, anti-degradants, U.V. stabilizers, coagents, comonomers, antistatic agents, water tree retarding agents, (silane) coupling agents, adhesion promotors, blowing agents, mold release agents, fillers, and process oils. These additives may be added in their usual amounts. For example, a liquid mixture of organic peroxide and unsaturated scorch retarder may be diluted on a filler, such as silica or calcium carbonate.

When a coagent (i.e., a chemical known to increase the crosslink density of a peroxide-cured polymer) is added, the organic peroxide formulations of the first aspect may contain from 40 to 70 wt.% of organic peroxide, from 3 to 30 wt.% of unsaturated scorch retarder, and from 15 to 50 wt.% of coagent, based on the total weight of the formulation, preferably from 45 to 65 wt.% of organic peroxide, from 5 to 25 wt.% of unsaturated scorch retarder, and from 20 to 40 wt.% of coagent, and more preferably from 50 to 60 wt.% of organic peroxide, from 10 to 20 wt.% of unsaturated scorch retarder, and from 25 to 35 wt.% of coagent. Any conventional crosslinking coagent may be used, with preferred coagents being selected from TAIC (triallyl isocyanurate), TAC (triallyl cyanurate), trimethylolpropane trimethacrylate, propoxylated 3-trimethylolpropane triacrylate, trimethylolpropane triacrylate, and mixtures thereof.

The organic peroxide formulations of the first aspect of the invention optionally may also contain another scorch retarder, i.e., a scorch retarder not having a structure of general formula (I), for example OH-Tempo. Such combinations of different scorch retarders have been found to cause a synergistic improvement to the scorch time.

The organic peroxide formulations of the first aspect of the invention preferably are used in crosslinking processes, more preferably, processes to crosslink polymers at elevated temperatures. In these processes, the organic peroxide formulation may be added to the polymer that is to be crosslinked by well-known methods (described below). To aid addition of the organic peroxide formulation to the polymer, the organic peroxide formulations according to the first aspect of the invention are preferably liquid at normal temperature and pressure (that is, at 20 °C and 101 kPa).

In a second aspect, the present disclosure is directed to a polymer composition comprising, consisting of, or consisting essentially of:
at least one organic peroxide;
at least one polymer; and
at least one unsaturated scorch retarder having the structure of general formula (I): 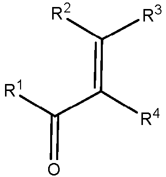 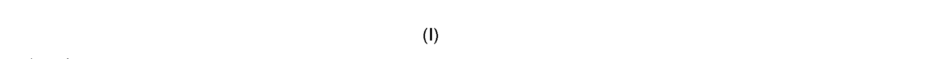 wherein,
R¹ is selected from OH, O-R⁵, or a saturated or unsaturated, linear, branched, or cyclic C₁ to C₈ alkyl group;
R² is on each occurrence independently selected from H or a saturated or unsaturated, linear or branched C₁ to C₈ alkyl group;
R³ is on each occurrence independently selected from H, O-R⁶, or a saturated or unsaturated, linear or branched C₁ to C₈ alkyl group;
R⁴ is on each occurrence independently selected from OH, O-R⁶, or a saturated or unsaturated, linear or branched C₁ to C₈ alkyl group;
R⁵ is selected from a saturated or unsaturated, linear or branched C₁ to C₈ alkyl group or -[CH₂]ₙ-OCOCR⁴CR²R³;
R⁶ is on each occurrence independently selected from a saturated or unsaturated, linear or branched C₁ to C₈ alkyl group; and
n is from 2-8, preferably from 3-6, and most preferably 6,
with the proviso that one but not both of R² and R³ are H,
   or wherein
R¹ and R² are joined to form a 5, 6, or 7 membered ring, said ring being formed of atoms selected from C, O, and S and containing one or two carbon-carbon double bonds and one or two carbonyl groups, said ring being optionally substituted with one or more saturated or unsaturated, linear or branched C₁ to C₈ alkyl group;
R³ is H; and
R⁴ and R⁶ are as described above,
   or wherein
R³ and R⁴ are joined to form a 5, 6, or 7 membered ring, said ring being formed of atoms selected from C, O, and S and containing one or two carbon-carbon double bonds, optionally substituted with one or more saturated or unsaturated, linear or branched C₁ to C₈ alkyl group;
R² is H; and
R¹, R⁵, R⁶ and n are as described above.

In this polymer composition, the at least one organic peroxide and the at least one unsaturated scorch retarder may be as defined above in the first aspect.

As used herein, the term "polymer" includes organic molecules with a weight average molecular weight higher than 10,000 g/mol, preferably 20,000 g/mol, and more preferably higher than 50,000 g/mol, as measured by gel permeation chromatography. The term "polymer" encompasses homopolymers and copolymers, where the term "copolymers" refers to a polymer comprised of at least two different monomers in polymerized form. For example, a copolymer in accordance with the present disclosure may be a polymer comprising two different monomers, a terpolymer is a polymer comprising three different monomers or more. The term "polymer" in this specification includes elastomers like ethylene-vinyl acetate (EVA) and polyolefin elastomers (POE), and synthetic rubbers like nitrile rubber (NBR), hydrogenated nitrile rubber (HNBR), and carboxylated nitrile butadiene rubber (XNBR).

The polymer composition of the second aspect of the invention preferably contains a polymer selected from ethylene-vinylacetate (EVA), low density polyethylene (LDPE), high density polyethylene (HDPE), polyolefin elastomers (POE), nitrile rubber (NBR), hydrogenated nitrile rubber (HNBR), carboxylated nitrile butadiene rubber (XNBR), and mixtures thereof. Preferably, the polymer is not ethylene propylene elastomers (EPM) or ethylene propylene diene elastomers (EPDM).

As indicated above, a mixture of two or more polymers can be used in the polymer composition of the second aspect. In this case, a mixture of ethylene-vinyl acetate (EVA) and polyolefin elastomers (POE) is preferred, in a weight ratio of EVA to POE of from 10:90 to 90:10. Advantageously, when a mixture of two or more polymers is used, the mixture may contain polymers with different melting points in order to tune their melting and processing behavior.

In a third aspect, the present disclosure is directed to a polymer composition comprising, consisting of, or consisting essentially of:
at least one organic peroxide;
at least one polymer selected from ethylene-vinylacetate (EVA), low density polyethylene (LDPE), high density polyethylene (HDPE), polyolefin elastomers (POE), nitrile rubber (NBR), hydrogenated nitrile rubber (HNBR), carboxylated nitrile butadiene rubber (XNBR), and mixtures thereof; and
at least one unsaturated scorch retarder having the structure of general formula (II): 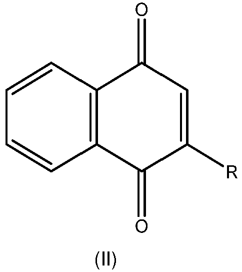 wherein
   R is selected from OH, O-R", or a saturated or unsaturated, linear or branched C₁ to C₈ alkyl group; and
   R" is a saturated or unsaturated, linear or branched C₁ to C₈ alkyl group.

The expression "saturated or unsaturated, linear or branched C₁ to C₈ alkyl group" has the meaning described above.

In a preferred embodiment of the third aspect, the at least one unsaturated scorch retarder has the structure of general formula (II), wherein:
R is selected from OH, O-R", or a saturated, linear C₁ to C₄ alkyl group; and
R" is a saturated, linear C₁ to C₄ alkyl group.

More preferably, R is selected from OH, O-Me, O-Et, methyl, ethyl, n-propyl or isopropyl. Most preferably, R is selected from OH or methyl. Preferred compounds with this structure are 2-methylnaphthalene-1,4-dione and 2-hydroxy-1,4-naphthoquinone.

In a fourth aspect, the present disclosure is directed to a polymer composition comprising, consisting of, or consisting essentially of:
at least one organic peroxide selected from tert-butylperoxy 2-ethylhexyl carbonate, tert-amylperoxy 2-ethylhexyl carbonate, and mixtures thereof;
1,1'-(1,1-dimethyl-3-methylene-1,3-propanediyl)bisbenzene (aMSD); and
at least one polymer selected from ethylene-vinylacetate (EVA), polyolefin elastomers (POE), and mixtures thereof.

Because aMSD is highly reactive, the polymer compositions of the fourth aspect of the present invention may contain as little as from 0.01 to 0.25 parts aMSD per hundred parts of polymer, preferably from 0.01 to 0.15 parts aMSD per hundred parts of polymer.

The polymer compositions of the second, third and fourth aspect of the present invention optionally may contain other additives, including crosslinking coagents (as defined above in the first aspect), antioxidants, heat stabilizers, U.V. stabilizers, (silane) coupling agents, adhesion promotors (e.g., 3-(trimethoxysilyl) propylmethacrylate), clarifiers, blowing agents, process oils, and fillers (including black, white, and colored fillers). These additives may be added in their usual amounts. For example, the polymer compositions may contain from 0.6 to 1 parts peroxide, from 0.3 to 0.7 parts coagent and from 0.1 to 0.3 parts adhesion promoter per hundred parts of polymer.

The polymer compositions of the second, third and fourth aspect of the present invention optionally may contain another scorch retarder, i.e., a scorch retarder not having a structure of general formula (I), general formula (II), or aMSD, for example OH-Tempo. Such combinations of different scorch retarders have been found to cause a synergistic effect on scorch.

The polymer(s), the peroxide(s), the scorch retarder(s) and all additives of the polymer compositions preferably have a purity of above 97%, more preferably above 98%. This allows them to be useful in solar or wire and cable applications.

The polymer compositions of the second, third and fourth aspect can be produced in a conventional manner. Typically, the polymer that is to be crosslinked is wetted or mixed with the organic peroxides and unsaturated scorch retarders (and optional other additives) at a temperature at or above room temperature and below the final cure temperature, using well-known equipment, such as a multi-roll mill (e.g., a two-roll mill), an extruder, a tumble mixer, or a calender assembly. The unsaturated scorch retarders act to prevent premature crosslinking during this mixing. The peroxide-containing polymer composition can be shaped using typical shaping processes such as injection molding (e.g., for shoe soles), sheet extrusion/calendering and compression molding (e.g., for EVA sheets for solar application), and extrusion and heat/infrared curing (e.g. for wire and cable application). Since shaping is a high-thermal process, which requires the peroxide-containing polymer compositions to flow well, the unsaturated scorch retarders are also critical to this step. After shaping, the peroxide-containing polymer composition is heated to effect final cure. For this stage, it is important not to have too high levels of unsaturated scorch retarders as this will slow the final crosslinking.

The polymer compositions of the second, third and fourth aspect of the present invention can be used to manufacture articles by curing the polymer composition. For example, the polymer compositions can be used to produce Solar Panel Encapsulant Sheets with increased scorch resistance, and hence improved processability during solar module production.

The invention will be elucidated by the following examples without being limited thereto or thereby.

### Examples

The unsaturated scorch retarders used in the following examples are as follows:
- Ethyl tiglate (ethyl trans-2-methyl-2-butenoate, TCI, > 98%)
- Tiglic acid (trans-2-methyl-2-butenoic acid, Sigma Aldrich, > 99%)
- L-Carvone (Sigma Aldrich, > 97%)
- D-Carvone (Natural Aroma India, > 95%)
- 1-Acetyl cyclohexene (Thermo Fisher Scientific, > 97%)
- Tiglate dimer (hexane-1,6-diyl(2E,2'E)-bis(2-methylbut-2-enoate), in-house, > 90%)
- Ketoisophorone (4-Oxoisophorone, Sigma Aldrich, 98%)
- 2,6-Dimethylbenzoquinone (TCI, 98%)
- 2-Hydroxy-1,4-naphthoquinone (Sigma Aldrich, 97%)
- Menadione (2-methylnaphthalene-1,4-dione, Sigma Aldrich, 98%)
- p-xyloquinone (2,5-dimethylbenzoquinone, TCI, 98%)
- 3-Methyl-2(*5H*)-furanone (Sigma Aldridge, 90%)
- 2-Methyl-2-cyclopentene-1-one (Sigma Aldridge, 98%).

Comparative scorch retarders used in the following examples are as follows:
- Ethyl 3,3-dimethacrylate (Sigma Aldrich, 98%)
- Ethyl crotonate (ethyl (E)-but-2-enoate, Sigma Aldrich, > 98%)
- Crotonic acid (trans-2-Butenoic acid, Sigma Aldrich, > 98%)
- Isophorone (3,5,5-Trimethylcyclohex-2-en-1-one, Sigma Aldrich, > 98%)
- R-Limonene (Sigma Aldrich, > 90%)
- Citraconimide dimer (1,1'-(hexane-1,6-diyl)bis(3-methyl-1H-pyrrole-2,5-dione, in-house, > 95%)
- Pulegone (Sigma Aldrich, > 90%)
- HEMA (Hydroxyethylmethacrylate, Sigma Aldrich, > 97%)
- OH-TEMPO (Eustab PI-172 ex Eutec chemical, > 99%).

### Preparation of EVA and POE-based polymer compositions

Unless otherwise stated, the EVA- and POE-based polymer compositions of the examples were prepared according to the following general procedure. This treatment assures that homogenous and reproducible samples are tested for cure performance and scorch delay.

EVA/POE granules (typical dimensions < 5 x 5 x 5 mm) were wetted with the indicated amount of liquid peroxide and liquid scorch retarder and then left in a closed glass jar to soak up the liquids, with intermittent stirring to homogenise. After full soaking of the granules (up to 24 hours) further homogenization was performed by treating the soaked granules on a two-roll mill at a temperature between 50 and 70°C. This produced a homogeneous sheet of the polymer, peroxide and scorch retarder.

In cases where the scorch retarder is a solid (e.g., menadione and OH-TEMPO) the solid scorch retarders are added on the two-roll-mill.

### Preparation of LDPE polymer compositions

BPD 2000: The BPD 2000 series granules (typical dimensions < 5 x 5 x 5 mm) were added to a glass jar and put in an air circulated oven at 60°C for 30 minutes. The granules were then soaked with the indicated amount of liquid peroxide and liquid scorch retarder at 60°C for 1 hour or until completely dry.

BPD 6107: The BPD 6170 powder was soaked with the indicated amount of liquid peroxide and liquid scorch retarder at room temperature for over 12 hours.

For all LDPE grades, the soaking process led to homogenous distribution of the additives over the LDPE granules.

### Scorch time measurements

Scorch time was determined by the Mooney scorch test (performed using Alpha Technologies Premier MV) and/or a rheometer (performed using Alpha Technologies Premier MDR).

As set out above, the Mooney scorch test (ISO 289-2:1994 (Mooney scorch)) describes two relevant parameters: "T5" and "T35", meaning the time to reach a 5 MU or a 35 MU higher readout of the Mooney viscosity, respectively. The "T5" parameter is a particularly good measure of the effect of scorch retarders. ("T35" is sometimes not possible to readout because some polymers are either too soft or become so brittle that the increase of 35 MU takes an inconvenient long time to measure, or never occurs).

The rheometer test is particularly useful when investigating melting polymers and thermoplastics. In a rheometer, the torque-time curve can be evaluated at a temperature, for example, 20-30 °C lower than the typical crosslink temperature (depending on melting point). From the values measured and the curve obtained, the effect of the scorch retarder on the safe processing times can be concluded.

Typical rheometer output data such as "ts1" and "ts2" can be found in the literature. These are normally interpreted as scorch parameters (with "s" standing for scorch). However, the meaning of these values is *"the time to reach a 0.1 Nm or 0.2 Nm higher torque",* respectively. This works as a scorch indicator if the final torque of the polymer composition is in the order of >2 Nm. However, for many unfilled polymers, these torques are not achieved. Thus, the following examples instead report the "t5" and "t10" times, i.e., the time required to reach 5 or 10% of the final cure level, respectively. As shown below, "t5" and "t10" have a good correlation with the Mooney scorch times.

The rheometer was also used to measure:
- ML, the minimum torque readout (viscosity minimum);
- MH, the maximum torque readout (viscosity maximum, i.e., crosslinking density); and
- Delta S, the difference between MH and ML.

Delta S is a good measure for the amount of crosslinking occurring in the tested sample.

### Example 1: Performance of unsaturated scorch retarders in peroxyketal-containing EVA composition

In this example, the scorch times of polymer compositions comprising EVA E282PV (random copolymer EVA with a VA content of 28% and a MFI of 25 g/10min) and Trigonox 29 (1, 1-di(tert-butylperoxy)-3,3,5-trimethylcyclohexane, Nouryon, 90%) were investigated with and without added unsaturated scorch retarder. The polymer compositions were made according to the general procedure described above.

Scorch time was determined using the Mooney scorch test and the rheometer test described above. The results are in Table 1. Here, L-Carvone was added to the composition in an equimolar amount vs tiglic acid.

**Table 1: Scorch times of peroxide-containing EVA composition with and without scorch retarder**

| | | | | | | | | |
|---|---|---|---|---|---|---|---|---|
| EVA E282PV | | 100 | 100 | 100 | 100 | 100 | 100 | 100 |
| Trigonox 29 | [phr] | 1 | 1 | 1 | 1 | 1 | 1 | 1 |
| Ethyl tiglate | [phr] | | 0.1 | 0.2 | 0.3 | | | |
| Tiglic acid | [phr] | | | | | 0.2 | | |
| Ethyl 3,3-dimethacrylate* | [phr] | | | | | | 0.2 | |
| L-Carvone | [phr] | | | | | | | 0.234 |
| | | | | | | | | |
| Rheometer 150 °C | | | | | | | | |
| Time | [min] | 30 | 30 | 30 | 30 | 30 | 30 | 30 |
| **t5** | [min] | **0.48** | **0.6** | **0.67** | **0.75** | **0.77** | **0.53** | **0.75** |
| **t10** | [min] | **0.57** | **0.74** | **0.87** | **0.98** | **1.01** | **0.65** | **0.99** |
| t50 | [min] | 1.47 | 1.93 | 2.35 | 2.75 | 2.92 | 1.75 | 2.85 |
| t90 | [min] | 5.34 | 6.34 | 7.43 | 8.58 | 8.78 | 6.11 | 8.39 |
| ML | [Nm] | 0.01 | 0.01 | 0.01 | 0.01 | 0.01 | 0.01 | 0.01 |
| MH | [Nm] | 0.22 | 0.21 | 0.20 | 0.18 | 0.20 | 0.19 | 0.22 |
| Delta S | [Nm] | 0.21 | 0.21 | 0.20 | 0.17 | 0.19 | 0.18 | 0.21 |
| | | | | | | | | |
| Mooney Scorch 120°C | | | | | | | | |
| **T5** | [min] | **7.78** | **12.34** | **17.09** | **21.05** | **22.46** | **10.85** | **22.28** |
| **T35** | [min] | **13.89** | **23.53** | **35.65** | **43.56** | **49.76** | **24.48** | **41.03** |

| | | | | | | | | |
|---|---|---|---|---|---|---|---|---|
| **Comparative* | | | | | | | | |

The results show that the addition of an unsaturated scorch retarder having the structure of general formula (I) to the peroxyketal-containing EVA composition significantly increases the scorch time. For example, when 0.2 phr tiglic acid or 0.234 phr L-Carvone is added to the composition, the T5 Mooney Scorch is over 2.8 times longer than when no scorch retarder is present, and over 2 times higher than when 0.2 phr of an alternative unsaturated scorch retarder is included (here, ethyl 3,3-dimethacrylate is not within the structure of general formula (I) since R⁴ cannot be H). The addition of 0.234 phr L-Carvone can also be seen to have no detrimental effect on the final cure (Delta S).

### Example 2: Performance of unsaturated scorch retarders in peroxymonocarbonate-containing EVA

In this example, the scorch times of polymer compositions comprising EVA E282PV and Trigonox 117 (tert-butylperoxy 2-ethylhexyl carbonate, Nouryon, 98%) were investigated with and without added unsaturated scorch retarder. The polymer compositions were made according to the general procedure above.

Scorch time was determined using the Mooney scorch test and the rheometer test described above. The results can be seen in Table 2. All comparisons are on equimolar basis vs ethyl tiglate.

**Table 2: Scorch times of peroxide-containing EVA composition with and without scorch retarder**

| | | | | | | | | |
|---|---|---|---|---|---|---|---|---|
| EVA E282PV | | 99 | 98.8 | 98.84 | 98.82 | 98.87 | 98.77 | 98.77 |
| Trigonox 117 | [%] | 1 | 1 | 1 | 1 | 1 | 1 | 1 |
| Ethyl tiglate | [%] | | 0.2 | | | | | |
| Tiglic acid | [%] | | | 0.156 | | | | |
| Ethyl crotonate* | [%] | | | | 0.178 | | | |
| Crotonic acid* | [%] | | | | | 0.134 | | |
| D-Carvone | [%] | | | | | | 0.234 | |
| L-Carvone | [%] | | | | | | | 0.234 |
| | | | | | | | | |
| Rheometer 150 °C | | | | | | | | |
| Time | [min] | 30 | 30 | 30 | 30 | 30 | 30 | 30 |
| **t5** | [min] | **0.66** | **1.12** | **1.13** | **0.83** | **0.79** | **1.29** | **1.28** |
| **t10** | [min] | **0.86** | **1.56** | **1.58** | **1.12** | **1.06** | **1.83** | **1.82** |
| t50 | [min] | 2.86 | 4.71 | 4.67 | 3.56 | 3.42 | 5.49 | 5.49 |
| t90 | [min] | 10.24 | 13.16 | 12.85 | 11.19 | 11.07 | 14.45 | 14.83 |
| ML | [Nm] | 0.01 | 0.01 | 0.01 | 0.01 | 0.01 | 0.01 | 0.01 |
| MH | [Nm] | 0.36 | 0.33 | 0.33 | 0.34 | 0.34 | 0.34 | 0.35 |
| Delta S | [Nm] | 0.35 | 0.32 | 0.32 | 0.33 | 0.33 | 0.33 | 0.34 |
| | | | | | | | | |
| Mooney scorch 120°C | | | | | | | | |
| **T5** | [min] | **12.1** | **29.98** | **27.87** | **16.77** | **19.83** | **39.16** | **38.19** |
| **T35** | [min] | **23.84** | **60.13** | **70.9** | **35.04** | **39.01** | **92.52** | **95.37** |

| | | | | | | | | |
|---|---|---|---|---|---|---|---|---|
| **Comparative* | | | | | | | | |

The results show that the addition of an unsaturated scorch retarder having the structure of general formula (I) to the peroxymonocarbonate-containing EVA composition significantly increases the scorch time. For example, when 0.234 % D-Carvone is added to the composition, the T5 Mooney Scorch is over 3.2 times longer than when no scorch retarder is present, and between 2-2.3 times higher than when an equimolar amount of an alternative unsaturated scorch retarder is used. Here, ethyl crotonate and crotonic acid are not within the structure of general formula (I) since R⁴ cannot be H.

### Example 3: Performance of L-Carvone in dialkyl peroxide-containing LDPE composition

In this example, the scorch times of polymer compositions comprising BPD2000 (LDPE with a MFI of 2 g/10min) and Trigonox T (tert-butyl cumyl peroxide, Nouryon, 95%) were investigated with and without added L-Carvone. The polymer compositions were made according to the general procedure above.

Scorch time was determined using the rheometer test described above (measured at 160 °C, i.e., 20 °C below the final crosslink temperature of 180 °C). The results can be seen in Table 3.

**Table 3: Scorch times of peroxide-containing LDPE composition with and without L-Carvone**

| | | | | | |
|---|---|---|---|---|---|
| BPD2000 | | 100 | 100 | 100 | 100 |
| Trigonox T | [phr] | 1.8 | 1.8 | 1.8 | 1.8 |
| L-Carvone | [phr] | | 0.1 | 0.2 | 0.4 |
| | | | | | |
| Rheometer 180°C | | | | | |
| Time | [min.] | 30 | 30 | 30 | 30 |
| t5 | [min.] | 0.74 | 0.8 | 0.83 | 0.98 |
| t10 | [min.] | 0.96 | 1.03 | 1.08 | 1.18 |
| t50 | [min.] | 2.98 | 3.01 | 3.07 | 3.36 |
| t90 | [min.] | 9.2 | 9.21 | 9.09 | 9.72 |
| delta S | [Nm] | 0.51 | 0.51 | 0.52 | 0.5 |
| | | | | | |
| Rheometer 160°C | | | | | |
| Time | [min.] | 60 | 60 | 60 | 30 |
| **t5** | [min.] | **1.55** | **2.5** | **3.05** | **3.37** |
| **t10** | [min.] | **2.84** | **4.09** | **4.8** | **5.56** |
| t50 | [min.] | 14.15 | 15.73 | 16.79 | 19.16 |
| t90 | [min.] | 41.26 | 42.12 | 42.65 | 44.22 |
| Delta S | [Nm] | 0.54 | 0.53 | 0.53 | 0.5 |

The results show that the addition of L-Carvone to the dialkyl peroxide-containing LDPE composition significantly increases the scorch time. For example, when 0.4 phr L-Carvone is added to the composition, the t5 is over 2.1 times longer than when no scorch retarder is present.

### Example 4: Performance of L-Carvone in peroxymonocarbonate-containing POE composition

In this example, the scorch times of polymer compositions comprising POE 8660 (ethylene - octene copolymer with a MFI of 4.8 g/10min) and Trigonox 131 (tert-amylperoxy 2-ethylhexyl carbonate, Nouryon, 97 %) were investigated with and without added L-Carvone. The polymer compositions were made according to the general procedure described above.

Scorch time was determined using the Mooney scorch test and the rheometer test described above. The results can be seen in Table 4.

**Table 4: Scorch times of peroxide-containing POE composition with and without L-Carvone**

| | | | | | |
|---|---|---|---|---|---|
| POE 8660 | | 100 | 100 | 100 | 100 |
| Trigonox 131 | [phr] | 1 | 1 | 1 | 1 |
| L-Carvone | [phr] | | 0.1 | 0.2 | 0.3 |
| | | | | | |
| Rheometer 150°C | | | | | |
| Time | [min.] | 30 | 30 | 30 | 30 |
| ts1 | [min.] | 2.74 | 2.47 | 2.73 | 2.99 |
| ts2 | [min.] | 10.27 | 5.65 | 5.96 | 6.79 |
| **t5** | [min.] | **0.72** | **0.82** | **0.88** | **0.92** |
| **t10** | [min.] | **0.93** | **1.08** | **1.18** | **1.25** |
| t50 | [min.] | 2.97 | 3.22 | 3.58 | 3.79 |
| t90 | [min.] | 8.97 | 9.12 | 9.67 | 10.18 |
| ML | [Nm] | 0.02 | 0.02 | 0.02 | 0.02 |
| MH | [Nm] | 0.23 | 0.28 | 0.29 | 0.28 |
| Delta S | [Nm] | 0.22 | 0.27 | 0.27 | 0.26 |
| | | | | | |
| Mooney scorch 110 °C | | | | | |
| **T5** | [min.] | **46.41** | **85.94** | **102.19** | **122.12** |
| **T35** | [min.] | **n.d.** | **n.d.** | **n.d.** | **n.d.** |

The results show that the addition of L-Carvone to the peroxymonocarbonate-containing POE composition significantly increases the scorch time. For example, when 0.3 phr L-Carvone is added, the T5 Mooney Scorch is over 2.6 times longer than when no scorch retarder is present. The addition of L-Carvone can also be seen to enhance the final cure (Delta S).

T35 could not be measured in this example because the viscosity build-up in the experiment never reached a level of 35 units above the minimum.

### Example 5: Performance of unsaturated scorch retarders in peroxymonocarbonate-containing EVA

In this example, the scorch times of polymer compositions comprising EVA E282PV and Trigonox 117 (Nouryon, 98 %) were investigated with and without added unsaturated scorch retarder. The polymer compositions were made according to the general procedure described above.

Scorch time was determined using the rheometer test described above. The results can be seen in Table 5. All comparisons are on equimolar basis vs D-carvone.

**Table 5: Scorch times of peroxide-containing EVA composition with and without scorch retarder**

| | | | | | | | |
|---|---|---|---|---|---|---|---|
| EVA E282PV | | 100 | 100 | 100 | 100 | 100 | 100 |
| Trigonox 117 | [phr] | 1 | 1 | 1 | 1 | 1 | 1 |
| Ketoisophorone | [phr] | | 0.2 | | | | |
| Isophorone* | [phr] | | | 0.18 | | | |
| D-carvone | [phr] | | | | 0.2 | | |
| 1-acetyl cyclohexene | [phr] | | | | | 0.165 | |
| R-Limonene* | [phr] | | | | | | 0.18 |
| | | | | | | | |
| Rheometer 150°C | | | | | | | |
| Time | [min.] | 30 | 30 | 30 | 30 | 30 | 30 |
| ts1 | [min.] | 1.37 | 4.19 | 2.02 | 3.04 | 3.2 | 2.06 |
| ts2 | [min.] | 2.84 | 7.09 | 4.86 | 5.33 | 6.78 | 4.01 |
| **t5** | [min.] | **0.66** | **1.68** | **0.74** | **1.21** | **1.05** | **0.84** |
| **t10** | [min.] | **0.82** | **2.35** | **0.96** | **1.7** | **1.5** | **1.15** |
| t50 | [min.] | 2.42 | 5.59 | 2.95 | 4.89 | 4.45 | 3.49 |
| t90 | [min.] | 9.61 | 13.38 | 10.17 | 12.95 | 12.48 | 10.91 |
| ML | [Nm] | 0.01 | 0.01 | 0.01 | 0.01 | 0.01 | 0.01 |
| MH | [Nm] | 0.37 | 0.32 | 0.29 | 0.37 | 0.29 | 0.36 |
| Delta S | [Nm] | 0.36 | 0.31 | 0.29 | 0.36 | 0.29 | 0.36 |
| | | | | | | | |
| Mooney scorch 120°C | | | | | | | |
| **T5** | [min.] | **10.87** | **72.76** | **17.01** | **34.19** | **34.95** | **19.58** |
| **T35** | [min.] | **21.66** | **> 120** | **44.92** | **78.85** | **98.37** | **39.89** |

| | | | | | | | |
|---|---|---|---|---|---|---|---|
| **Comparative* | | | | | | | |

The addition of an unsaturated scorch retarder having the structure of general formula (I) to the peroxymonocarbonate-containing EVA composition can be seen to significantly increase the scorch time. For example, when 0.2 phr ketoisophorone is added, the T5 Mooney Scorch is over 6.6 times longer than when no scorch retarder is present, and between 3.7-4.3 times higher than when an equimolar amount of an alternative unsaturated scorch retarder is used (here, isophorone is not within the structure of general formula (I) since R⁴ cannot be H, and R-Limonene is not within the structure of general formula (I) since it lacks a carbonyl group).

### Example 6: Performance of unsaturated scorch retarders in peroxymonocarbonate-containing EVA

In this example, the scorch times of polymer compositions comprising PV1400 EVA (photovoltaic grade EVA copolymer with 33 wt% VA) and Trigonox 117 (Nouryon, 98 %) were investigated with and without added unsaturated scorch retarder. The polymer compositions were made according to the general procedure described above.

Scorch time was determined using the Mooney scorch test and the rheometer test described above. The results can be seen in Table 6. All comparisons are on equimolar basis vs ketoisophorone.

**Table 6: Scorch times of peroxide-containing EVA composition with and without scorch retarders**

| PV1400 EVA | | 100 | 100 | 100 | 100 | 100 |
|---|---|---|---|---|---|---|
| Trigonox 117 | [phr] | 1 | 1 | 1 | 1 | 1 |
| Ketoisophorone | [phr] | | 0.1 | | | |
| 2,6-Dimethylbenzoquinone | [phr] | | | 0.09 | | |
| 2-Hydroxy-1,4-naphthoquinone | [phr] | | | | 0.114 | |
| Menadione | [phr] | | | | | 0.113 |
| | | | | | | |

| Rheometer 150°C | | | | | | |
|---|---|---|---|---|---|---|
| Time | [min.] | 30 | 30 | 30 | 30 | 30 |
| ts1 | [min.] | 1.41 | 2.87 | 3.02 | 4.48 | 3.96 |
| ts2 | [min.] | 2.92 | 4.91 | 4.87 | 7.59 | 6.16 |
| **t5** | [min.] | **0.68** | **1.42** | **1.68** | **1.99** | **2.05** |
| **t10** | [min.] | **0.85** | **1.84** | **2.06** | **2.63** | **2.62** |
| t50 | [min.] | 2.43 | 4.14 | 4.36 | 5.96 | 5.43 |
| t90 | [min.] | 9.12 | 11.39 | 12.12 | 13.91 | 13.01 |
| ML | [Nm] | 0 | 0 | 0 | 0 | 0 |
| MH | [Nm] | 0.36 | 0.34 | 0.36 | 0.31 | 0.35 |
| delta S | [Nm] | 0.35 | 0.34 | 0.36 | 0.31 | 0.34 |
| | | | | | | |
| Mooney scorch 120 °C | | | | | | |
| **T5** | [min] | **14** | **49** | **59** | **72** | **67** |
| **T35** | [min] | **26** | **71** | **104** | **>> 120** | **>>120** |

The results show that unsaturated scorch retarder with a "dione" structure are very good scorch retarders in EVA.

### Example 7: Performance of Ketoisophorone in dialkyl peroxide-containing LDPE composition

In this example, the scorch times of polymer compositions comprising BPD 6170 (a powdered grade low density LDPE) and Trigonox T (Nouryon, 95 %) were investigated with and without added ketoisophorone. The polymer compositions were made according to the general procedure described above.

Scorch time was determined using the rheometer test described above (measured at 160 °C, i.e., 20 °C below the final crosslink temperature of 180 °C). The results can be seen in Table 7.

**Table 7: Scorch times of peroxide-containing LDPE composition with and without ketoisophorone**

| | | | |
|---|---|---|---|
| LDPE BPD 6170 | | 100 | 100 |
| Trigonox T | [phr] | 1.8 | 1.8 |
| Ketoisophorone | [phr] | | 0.2 |
| | | | |

| Rheometer 180°C | | | |
|---|---|---|---|
| Time | [min.] | 30 | 30 |
| ts1 | [min.] | 1.05 | 1.12 |
| ts2 | [min.] | 1.59 | 1.61 |
| t5 | [min.] | 0.71 | 0.79 |
| t10 | [min.] | 0.94 | 1.01 |
| t50 | [min.] | 2.87 | 2.75 |
| t90 | [min.] | 8.63 | 8.11 |
| ML | [Nm] | 0 | 0 |
| MH | [Nm] | 0.79 | 0.77 |
| delta S | [Nm] | 0.78 | 0.77 |
| | | | |

| Rheometer 160°C | | | |
|---|---|---|---|
| Time | [min.] | 60 | 60 |
| ts1 | [min.] | 4.11 | 4.97 |
| ts2 | [min.] | 7.07 | 7.85 |
| **t5** | [min.] | **2.21** | **2.84** |
| **t10** | [min.] | **3.58** | **4.4** |
| t50 | [min.] | 14.74 | 14.97 |
| t90 | [min.] | 41.21 | 41.15 |
| ML | [Nm] | 0 | 0 |
| MH | [Nm] | 0.82 | 0.82 |
| Delta S | [Nm] | 0.82 | 0.82 |

The results show that the addition of ketoisophorone to the dialkyl peroxide-containing LDPE composition significantly increases the scorch time. Specifically, when 0.2 phr ketoisophorone is added to the composition, the t5 is over 1.2 times longer than when no scorch retarder is present.

### Example 8: Performance of unsaturated scorch retarders in peroxymonocarbonate-containing EVA

In this example, the scorch times of polymer compositions comprising EVA 360A (EVA copolymer with a VA content of 25 wt.% and MFI of 2 g/10min) and Trigonox 117 (Nouryon, 98 %) were investigated with and without added unsaturated scorch retarder. The polymer compositions were made according to the general procedure described above.

Scorch time was determined using the Mooney scorch test and the rheometer test described above. The results can be seen in Table 8. Here, citraconimide dimer was added on an equimolar basis vs tiglate dimer. The remaining comparisons are on equimolar basis vs D-carvone.

**Table 8: Scorch times of peroxide-containing EVA composition with and without scorch retarder**

| EVA 360A | | 100 | 100 | 100 | 100 | 100 | 100 | 100 | 100 | 100 |
|---|---|---|---|---|---|---|---|---|---|---|
| Trigonox 117 | [phr] | 1 | 1 | 1 | 1 | 1 | 1 | 1 | 1 | 1 |
| Tiglate dimer | [phr] | | 0.2 | | | | | | | |
| Citraconimide dimer* | [phr] | | | 0.22 | | | | | | |
| HEMA* | [phr] | | | | 0.09 | | | | | |
| Pulegone* | [phr] | | | | | 0.1 | | | | |
| ethyl tiglate | [phr] | | | | | | 0.084 | | | |
| D-carvone | [phr] | | | | | | | 0.1 | | |
| p-xyloquinone | [phr] | | | | | | | | 0.09 | |
| Ketoisophorone | [phr] | | | | | | | | | 0.1 |
| | | | | | | | | | | |

| Rheometer 150°C | | | | | | | | | | |
|---|---|---|---|---|---|---|---|---|---|---|
| Time | [min.] | 30 | 30 | 30 | 30 | 30 | 30 | 30 | 30 | 30 |
| ts1 | [min.] | 1.24 | 1.79 | 1.57 | 1.55 | 1.53 | 1.52 | 1.9 | 2.53 | 2.43 |
| ts2 | [min.] | 1.99 | 2.89 | 2.41 | 2.29 | 2.62 | 2.47 | 3.11 | 3.74 | 3.63 |
| **t5** | [min.] | **0.75** | **0.98** | **0.94** | **1** | **0.8** | **0.85** | **0.97** | **1.44** | **1.24** |
| **t10** | [min.] | **0.94** | **1.28** | **1.18** | **1.19** | **1.03** | **1.09** | **1.33** | **1.88** | **1.71** |
| t50 | [min.] | 2.65 | 3.67 | 3.04 | 2.67 | 3.09 | 3.07 | 3.93 | 4.47 | 4.18 |
| t90 | [min.] | 9.43 | 11.02 | 9.28 | 7.64 | 9.93 | 9.34 | 11.01 | 11.32 | 10.93 |
| ML | [Nm] | 0.04 | 0.03 | 0.04 | 0.04 | 0.04 | 0.04 | 0.04 | 0.04 | 0.04 |
| MH | [Nm] | 0.56 | 0.55 | 0.56 | 0.52 | 0.51 | 0.54 | 0.56 | 0.54 | 0.52 |
| Delta S | [Nm] | 0.52 | 0.52 | 0.52 | 0.48 | 0.47 | 0.5 | 0.52 | 0.5 | 0.48 |
| | | | | | | | | | | |
| Mooney Scorch 120 °C | | | | | | | | | | |
| **T5** | [min.] | **17.34** | **25.52** | **16.91** | **20.25** | **20.76** | **22.17** | **30.55** | **36.07** | **49.81** |
| **T35** | [min.] | **32.81** | **62.15** | **41.34** | **34.16** | **52.75** | **46.84** | **71.13** | **72.52** | **85.36** |

| | | | | | | | | | | |
|---|---|---|---|---|---|---|---|---|---|---|
| **Comparative* | | | | | | | | | | |

The results show that unsaturated compounds having the structure of general formula (I) are very good scorch retarders in EVA. In contrast, HEMA (a compound in which R² and R³ are H) and pulegone (a compound in which neither R² nor R³ is H) are shown to only marginally increase the T5 Mooney Scorch, while diminishing the final cure. Likewise, the citraconimide dimer (a compound in which R¹ and R² are joined to form a N-containing ring) can be seen to have very little effect on the T5 and T35 Mooney Scorch compared to the tiglate dimer.

### Example 9: Scorch performance of protected organic peroxide formulation

In this example, the scorch times of polymer compositions comprising PV1400 EVA and Trigonox 117 (Nouryon, 98 %) were investigated with and without added unsaturated scorch retarder. The polymer compositions were made by wetting the EVA granules, as explained in the general method set out above, with an organic peroxide formulation selected from:
(i) a blended, liquid-liquid mixture of 90% Trigonox 117 and 10% ketoisophorone, or
(ii) a blended, liquid-liquid mixture of 90% Trigonox 117 and 10% OH-TEMPO.

Scorch time was determined using the Mooney scorch test and the rheometer test described above. The results can be seen in Table 9.

**Table 9: Scorch times of peroxide-containing EVA composition with and without scorch retarders**

| PV1400 EVA | | 100 | 100 | 100 |
|---|---|---|---|---|
| Trigonox 117 | | 1 | | |
| Trigonox 117 incl 10% ketoisophorone | | | 1.11 | |
| Trigonox 117 incl 10% OH-TEMPO* | | | | 1.11 |
| | | | | |

| Rheometer 150°C | | | | |
|---|---|---|---|---|
| Time | [min] | 30 | 30 | 30 |
| ts1 | [min.] | 1.41 | 3 | 2.38 |
| ts2 | [min.] | 2.92 | 4.99 | 4.4 |
| **t5** | [min.] | **0.68** | **1.51** | **1.39** |
| **t10** | [min.] | **0.85** | **1.94** | **1.61** |
| t50 | [min.] | 2.43 | 4.18 | 3.36 |
| t90 | [min.] | 9.12 | 11.13 | 10.4 |
| ML | [Nm] | 0 | 0 | 0 |
| MH | [Nm] | 0.36 | 0.34 | 0.32 |
| Delta S | [Nm] | 0.35 | 0.33 | 0.32 |
| | | | | |

| Mooney scorch 120 °C | | | | |
|---|---|---|---|---|
| **T5** | [min] | **14** | **56** | **43** |
| **T35** | [min] | **26** | **83** | **57** |

| | | | | |
|---|---|---|---|---|
| **Comparative* | | | | |

The results show that organic peroxide formulations containing an unsaturated scorch retarder with the structure of general formula (I) provide improved scorch protection in EVA over the commercially available OH-TEMPO.

### Example 10: Scorch performance of a-MSD in peroxymonocarbonate-containing EVA composition

In this example, the scorch times of polymer compositions comprising EVA E282PV and Trigonox 117 (Nouryon, 98 %) were investigated with and without added a-MSD. The polymer compositions were made according to the general procedure described above.

Scorch time was determined using the Mooney scorch test and the rheometer test described above. The results can be seen in Table 10.

**Table 10: Scorch times of peroxide-containing EVA composition with and without a-MSO**

| EVA E282PV | | 100 | 100 | 100 | 100 |
|---|---|---|---|---|---|
| Trigonox 117 | [phr] | 1 | 1 | 1 | 1 |
| a-MSD | [phr] | | 0.1 | 0.2 | 0.3 |
| | | | | | |

| Rheometer 150°C | | | | | |
|---|---|---|---|---|---|
| Time | [min.] | 30 | 30 | 30 | 30 |
| ts1 | [min.] | 1.45 | 3.17 | 4.36 | 6.31 |
| ts2 | [min.] | 3 | 5.09 | 7.37 | 13.9 |
| **t5** | [min.] | **0.69** | **1.4** | **1.65** | **1.74** |
| **t10** | [min.] | **0.86** | **1.92** | **2.39** | **2.56** |
| t50 | [min.] | 2.47 | 4.76 | 6.49 | 7.67 |
| t90 | [min.] | 9.54 | 12.56 | 15.42 | 18.4 |
| ML | [Nm] | 0.01 | 0.01 | 0.01 | 0.01 |
| MH | [Nm] | 0.36 | 0.38 | 0.35 | 0.26 |
| Delta S | [Nm] | 0.35 | 0.37 | 0.35 | 0.25 |
| | | | | | |

| Mooney scorch 120°C | | | | | |
|---|---|---|---|---|---|
| **T5** | [min.] | **12.28** | **43.9** | **56.55** | **71.22** |
| **T35** | [min.] | **24.38** | **83.01** | **151.42** | **329.65** |

The results show that the addition of a-MSD to the peroxymonocarbonate-containing EVA composition significantly increases the scorch time. For example, when 0.3 phr a-MSD is added to the composition, the T5 Mooney Scorch is over 5.7 times longer than when no scorch retarder is present.

### Example 11: Scorch performance of menadione and OH-TEMPO in peroxymonocarbonate-containing EPDM or EVA compositions

In this example, the scorch times of polymer compositions comprising Trigonox 117 (Nouryon, 98%) and PV1400 EVA or EPDM (Keltan^{®} 5531A, Mooney viscosity ML(1+4) @ 125°C MU = 52, Ethylene content 63%, ENB: 4.5%, extender oil 50.0 wt.%) were investigated with and without added menadione or OH-TEMPO (in amounts based on peroxide addition). The polymer compositions were made using a two-roll mill operated at 50 °C. For EVA, the granules were pre-soaked with peroxide and menadione/OH-TEMPO was added (as a solid) on the two-roll mill. For EPDM, all additives were added on the two-roll mill.

Scorch time was determined using the above-described Mooney scorch test and rheometer test (measured at 150 °C, i.e., 30 °C below the final crosslink temperature of 180 °C). The results are in Table 11.

**Table 11: Scorch times of peroxide-containing EVA/EPDM with and without scorch retarders**

| | | | | | | | |
|---|---|---|---|---|---|---|---|
| EVA PV1400 | | 100 | 100 | 100 | | | |
| EPDM 5531A* | | | | | 100 | 100 | 100 |
| Trigonox 117 | [phr] | 1 | 1 | 1 | 2 | 2 | 2 |
| Menadione | [phr] | | 0.11 | | | 0.22 | |
| OH-TEMPO* | [phr] | | | 0.11 | | | 0.22 |
| | | | | | | | |
| Rheometer 150°C | | | | | | | |
| Time | [min.] | 30 | 30 | 30 | 30 | 30 | 30 |
| ts1 | [min.] | 1.52 | 4.27 | 2.43 | 1.49 | 1.6 | 1.91 |
| ts2 | [min.] | 2.85 | 6.68 | 4.23 | 4.26 | 3.89 | 4.62 |
| **t5** | [min.] | **0.8** | **2.18** | **1.42** | **0.56** | **0.58** | **0.84** |
| **t10** | [min.] | **0.97** | **2.76** | **1.67** | **0.69** | **0.76** | **1** |
| t50 | [min.] | 2.51 | 5.74 | 3.48 | 2.2 | 2.37 | 2.7 |
| t90 | [min.] | 8.83 | 13.28 | 10.55 | 9.17 | 9.13 | 9.33 |
| ML | [Nm] | 0 | 0 | 0 | 0.14 | 0.14 | 0.12 |
| MH | [Nm] | 0.37 | 0.34 | 0.34 | 0.42 | 0.43 | 0.4 |
| Delta S | [Nm] | 0.36 | 0.33 | 0.34 | 0.28 | 0.29 | 0.28 |
| | | | | | | | |
| Rheometer 180°C | | | | | | | |
| Time | [min.] | 10 | 10 | 10 | 10 | 10 | 10 |
| ts1 | [min.] | 0.47 | 0.62 | 0.56 | 0.45 | 0.49 | 0.47 |
| ts2 | [min.] | 0.66 | 0.89 | 0.85 | 0.88 | n.d | 0.91 |
| t5 | [min.] | 0.31 | 0.42 | 0.38 | 0.26 | 0.27 | 0.29 |
| t10 | [min.] | 0.35 | 0.47 | 0.42 | 0.29 | 0.3 | 0.32 |
| t50 | [min.] | 0.54 | 0.68 | 0.62 | 0.47 | 0.48 | 0.5 |
| t90 | [min.] | 0.98 | 1.21 | 1.21 | 0.88 | 0.9 | 0.91 |
| ML | [Nm] | 0 | 0 | 0 | 0.14 | 0.13 | 0.12 |
| MH | [Nm] | 0.3 | 0.27 | 0.26 | 0.36 | 0.33 | 0.34 |
| Delta S | [Nm] | 0.29 | 0.27 | 0.26 | 0.22 | 0.2 | 0.22 |
| | | | | | | | |
| Mooney scorch 120 °C | | | | | | | |
| **T5** | [min.] | **23.26** | **72.53** | **53.66** | **10.64** | **11.92** | **28.95** |
| **T35** | [min.] | **40.23** | >> **120** | **78.56** | > **120** | > **120** | **> 120** |

| | | | | | | | |
|---|---|---|---|---|---|---|---|
| **Comparative* | | | | | | | |

In Table 11, ts1 and ts2 (indicating a viscosity increase of 0.1 and 0.2 Nm, respectively) are not proper indicators of scorch times. This is because the overall crosslink density (MH) of the unfilled compounds only reaches 0.3 Nm. Instead, the Mooney scorch times should be compared. From these, it can be seen that menadione provides no real scorch protection in EPDM. However, in EVA, the addition of menadione (i.e., an unsaturated scorch retarder with the structure of general formula (II)) provides improved scorch protection over the commercially available OH-TEMPO.

### Example 12: Effect of coagent on scorch properties of peroxymonocarbonate-containing POE composition

The scorch times of polymer compositions comprising POE 8660 and Trigonox 117 (Nouryon, 98 %) or Trigonox 131 (Nouryon, 97 %) were investigated with and without added TAIC and/or D-Carvone (in amounts based on peroxide addition). The polymer compositions were made using a two-roll mill operated at 50-70 °C. The POE granules were pre-soaked with peroxide and TAIC and/or D-Carvone at room temperature for 18 hours.

Scorch time was determined using the above-described Mooney scorch test and rheometer test (measured at 150 °C). The results can be seen in Table 12.

**Table 12: Scorch times of peroxymonocarbonate-containing POE with and without TAIC/D-Carvone**

| | | | | | | | | | |
|---|---|---|---|---|---|---|---|---|---|
| POE 8660 | | 100 | 100 | 100 | 100 | 100 | 100 | 100 | 100 |
| Trigonox 131 | [phr] | 1 | 1 | 1 | 1 | | | | |
| Trigonox 117 | [phr] | | | | | 1 | 1 | 1 | 1 |
| TAIC | [phr] | | 0.5 | | 0.5 | | 0.5 | | 0.5 |
| D-Carvone | [phr] | | | 0.2 | 0.2 | | | 0.2 | 0.2 |
| | | | | | | | | | |
| Rheometer 150°C | | | | | | | | | |
| Time req. | [min] | 30 | 30 | 30 | 30 | 30 | 30 | 30 | 30 |
| ts1 | [min] | 2.9 | 0.91 | 2.57 | 1.5 | 2.5 | 1.22 | 3 | 2.15 |
| ts2 | [min] | 13.67 | 1.49 | 5.24 | 2.44 | 5.34 | 1.95 | 6.15 | 3.7 |
| **t5** | [min] | **0.72** | **0.59** | **0.9** | **0.81** | **0.86** | **0.77** | **0.98** | **1.02** |
| **t10** | [min] | **0.94** | **0.68** | **1.2** | **1.04** | **1.17** | **0.93** | **1.37** | **1.4** |
| t50 | [min] | 3.04 | 1.46 | 3.45 | 2.73 | 3.98 | 2.45 | 4.62 | 4.36 |
| t90 | [min] | 9.13 | 5.6 | 9.18 | 7.87 | 12 | 9.5 | 12.93 | 12.89 |
| ML | [Nm] | 0.02 | 0.02 | 0.02 | 0.02 | 0.02 | 0.02 | 0.02 | 0.02 |
| MH | [Nm] | 0.23 | 0.41 | 0.3 | 0.47 | 0.34 | 0.51 | 0.33 | 0.49 |
| **Delta S** | [Nm] | **0.21** | **0.39** | **0.28** | **0.45** | **0.32** | **0.49** | **0.32** | **0.47** |
| | | | | | | | | | |
| Mooney scorch 120 °C | | | | | | | | | |
| **T5** | [min] | **21** | **10** | **30** | **20** | **32** | **16** | **50** | **34** |
| **Δ T5** | [%] | | | **+43** | **+100** | | | **+56** | **+113** |
| **T35** | [min] | **> 120** | **15** | **> 120** | **60** | **> 120** | **33** | **> 120** | **> 120** |

Here, Δ T5 refers to the difference in T5 measurements upon adding a scorch retarder as a percentage of the T5 of the equivalent polymer composition without a scorch retarder (e.g., ((30-21)/21) x 100 = 43 and ((20-10)/10) x 100 = 100). From these values it can be seen that the effect of the scorch retarder is enhanced by the coagent (i.e., there is synergy).

The results show that the addition of TAIC to the peroxymonocarbonate-containing POE compositions significantly enhances final cure but results in a much shorter scorch time (giving rise to processing issues). In contrast, the combination of D-Carvone and TAIC can be seen to provide a similar increase in crosslinking density, but without compromising scorch safety. The addition of D-Carvone and TAIC to the Trigonox 131-containing POE composition can also be seen to reduce the crosslink time (t90) from 9.13 min to 7.87 min. Thus, the unsaturated scorch retarders can be used with coagents to provide a synergistic effect and balance all relevant crosslink performance parameters like time to cure, scorch safety and ultimate crosslink density.

### Example 13: Performance of unsaturated cyclic scorch retarders in peroxymonocarbonate-containing EVA

In this example, the scorch times of polymer compositions comprising EVA E282PV and Trigonox 117 (Nouryon, 98 %) were investigated with and without added unsaturated cyclic scorch retarder. The polymer compositions were made according to the general procedure described above.

Scorch time was determined using the Mooney scorch test and the rheometer test described above. The results can be seen in Table 13. All comparisons are on equimolar basis vs D-carvone.

**Table 13: Scorch times of peroxide-containing EVA composition with and without cyclic scorch retarder**

| | | | | | |
|---|---|---|---|---|---|
| EVA E282PV | | 100 | 100 | 100 | 100 |
| Trigonox 117 | [phr] | 1 | 1 | 1 | 1 |
| D-carvone | [phr] | | 0.2 | | |
| 3-Methyl-2(*5H*)-furanone | [phr] | | | 0.131 | |
| 2-Methyl-2-cyclopentene-1-one | [phr] | | | | 0.128 |
| | | | | | |
| Rheometer 150°C | | | | | |
| Time | [min.] | 30 | 30 | 30 | 30 |
| ts1 | [min.] | 1.45 | 3.23 | 2.72 | 3.01 |
| ts2 | [min.] | 3 | 5.83 | 4.87 | 5.36 |
| **t5** | **[min.]** | **0.69** | **1.23** | **1.11** | **1.18** |
| **t10** | **[min.]** | **0.86** | **1.74** | **1.51** | **1.64** |
| t50 | [min.] | 2.47 | 5.07 | 4.05 | 4.5 |
| t90 | [min.] | 9.54 | 13.48 | 11.35 | 12.17 |
| ML | [Nm] | 0.01 | 0.01 | 0.01 | 0.01 |
| MH | [Nm] | 0.36 | 0.36 | 0.34 | 0.34 |
| Delta S | [Nm] | 0.35 | 0.35 | 0.34 | 0.34 |
| | | | | | |
| Mooney Scorch 120 °C | | | | | |
| **T5** | **[min.]** | **12.28** | **35.94** | **28.16** | **28.9** |
| **T35** | **[min.]** | **24.38** | **86.49** | **55.52** | **54.57** |

The results show that the addition of an unsaturated cyclic scorch retarder having the structure of general formula (I) to the peroxymonocarbonate-containing EVA composition significantly increases the scorch time. For example, when 0.128 phr 2-Methyl-2-cyclopentene-1-one is added to the composition, the T5 Mooney Scorch is over 2.3 times longer than when no scorch retarder is present.

Whilst the invention has been described with reference to an exemplary embodiment, it will be appreciated that various modifications are possible within the scope of the invention.

In this specification, unless expressly otherwise indicated, the word 'or' is used in the sense of an operator that returns a true value when either or both of the stated conditions is met, as opposed to the operator 'exclusive or' which requires that only one of the conditions is met. The word 'comprising' is used in the sense of 'including' rather than to mean 'consisting of'. All prior teachings acknowledged above are hereby incorporated by reference. No acknowledgement of any prior published document herein should be taken to be an admission or representation that the teaching thereof was common general knowledge in Europe or elsewhere at the date hereof.

## Claims

1. An organic peroxide formulation comprising:
at least one organic peroxide; and
at least one unsaturated scorch retarder having the structure of general formula (I): 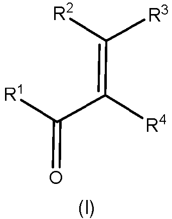 wherein,
R¹ is selected from OH, O-R⁵, or a saturated or unsaturated, linear, branched, or cyclic C₁ to C₈ alkyl group;
R² is on each occurrence independently selected from H or a saturated or unsaturated, linear or branched C₁ to C₈ alkyl group;
R³ is on each occurrence independently selected from H, O-R⁶, or a saturated or unsaturated, linear or branched C₁ to C₈ alkyl group;
R⁴ is on each occurrence independently selected from OH, O-R⁶, or a saturated or unsaturated, linear or branched C₁ to C₈ alkyl group;
R⁵ is selected from a saturated or unsaturated, linear or branched C₁ to C₈ alkyl group or -[CH₂]ₙ-OCOCR⁴CR²R³;
R⁶ is on each occurrence independently selected from a saturated or unsaturated, linear or branched C₁ to C₈ alkyl group; and
n is from 2-8,
with the proviso that one but not both of R² and R³ are H,
or wherein
R¹ and R² are joined to form a 5, 6, or 7 membered ring, said ring being formed of atoms selected from C, O, and S and containing one or two carbon-carbon double bonds and one or two carbonyl groups, said ring being optionally substituted with one or more saturated or unsaturated, linear or branched C₁ to C₈ alkyl group;
R³ is H; and
R⁴ and R⁶ are as described above,
or wherein
R³ and R⁴ are joined to form a 5, 6, or 7 membered ring, said ring being formed of atoms selected from C, O, and S and containing one or two carbon-carbon double bonds, said ring optionally substituted with one or more saturated or unsaturated, linear or branched C₁ to C₈ alkyl group;
R² is H; and
R¹, R⁵, R⁶ and n are as described above.

2. An organic peroxide formulation as claimed in claim 1, wherein
R¹ is selected from OH, O-R⁵, or a saturated, linear C₁ to C₄ alkyl group;
R² is on each occurrence independently selected from H or a saturated, linear C₁ to C₄ alkyl group;
R³ is on each occurrence independently selected from H, O-R⁶, or a saturated, linear C₁ to C₃ alkyl group;
R⁴ is on each occurrence independently selected from OH, O-R⁶, or a saturated, linear C₁ to C₄ alkyl group;
R⁵ is selected from a saturated, linear C₁ to C₄ alkyl group or -[CH₂]ₙ-OCOCR⁴CR²R³;
R⁶ is on each occurrence independently a saturated, linear C₁ to C₄ alkyl group; and
n is from 3-6,
with the proviso that one but not both of R² and R³ are H,
or wherein
R³ and R⁴ are joined to form a 6 membered ring, said ring being formed of atoms selected from C, O, and S and containing one or two carbon-carbon double bonds, said ring optionally substituted with one or more saturated, linear C₁ to C₄ alkyl group;
R² is H; and
R¹, R⁵, R⁶ and n are as described above.

3. An organic peroxide formulation as claimed in claim 1, wherein
R¹ and R² are joined to form a 5 or 6 membered ring, said ring being formed of atoms selected from C, O, and S and containing one or two carbon-carbon double bonds and one or two carbonyl groups, said ring being optionally substituted with one or more saturated or unsaturated, linear or branched C₁ to C₄ alkyl group;
R³ is H;
R⁴ is selected from OH, O-R⁶, or a saturated, linear C₁ to C₄ alkyl group; and
R⁶ is on each occurrence independently a saturated, linear C₁ to C₄ alkyl group.

4. An organic peroxide formulation as claimed in claim 1, comprising at least one unsaturated scorch retarder having the structure of general formula (IA) or (IB): 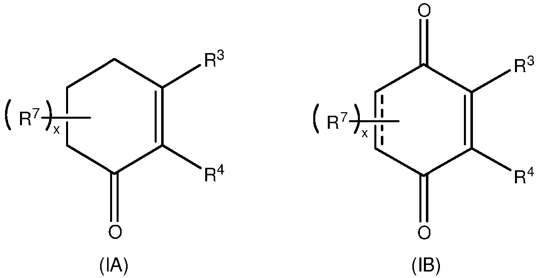 wherein
R³ is H;
R⁴ is selected from OH, O-R⁶, or a saturated, linear C₁ to C₄ alkyl group;
R⁶ is on each occurrence independently a saturated, linear C₁ to C₄ alkyl group;
R⁷ is on each occurrence independently selected from OH, O-R⁶ or a saturated or unsaturated, linear or branched C₁ to C₈ alkyl group;
x is from 0-4; and
the dotted line in formula (IB) is an optional double bond.

5. An organic peroxide formulation as claimed in any one of the preceding claims, wherein the at least one unsaturated scorch retarder is selected from ethyl trans-2-methyl-2-butenoate, *trans-*2-methyl-2-butenoic acid, hexane-1,6-diyl (2E,2'E)-bis(2-methylbut-2-enoate), 1-acetyl cyclohexene, L-carvone, D-carvone, 3-methyl-2(*5H*)-furanone, 2-methyl-2-cyclopenten-1-one, 2,6-dimethylbenzoquinone, 2,5-dimethylbenzoquinone, and ketoisophorone.

6. An organic peroxide formulation as claimed in any one of the preceding claims, that is liquid at normal temperature and pressure.

7. An organic peroxide formulation as claimed in any one of the preceding claims, comprising from 70 to 95 wt.% of the at least one organic peroxide and from 5 to 30 wt.% of the at least one scorch retarder, based on the total weight of the formulation.

8. An organic peroxide formulation as claimed in any one of claims 1 to 6, comprising from 40 to 70 wt.% of the at least one organic peroxide, from 3 to 30 wt.% of the at least one unsaturated scorch retarder, and from 15 to 50 wt.% of at least one crosslinking coagent, based on the total weight of the formulation.

9. An organic peroxide formulation as claimed in any one of the preceding claims, wherein the at least one organic peroxide is selected from tert-butyl peroxy 2-ethylhexyl carbonate, tert-amyl peroxy 2-ethylhexyl carbonate, tert-butyl peroxy isopropyl carbonate, 1,1-di(tert-butylperoxy)-3,3,5-trimethylcyclohexane, 1,1-di(tert-butylperoxy)-cyclohexane, butyl 4,4-di(tert-butylperoxy)valerate, tert-butyl peroxybenzoate, tert-butyl cumyl peroxide, 2,5-dimethyl-2,5-di(tert-butylperoxy)hexane, 2-5-dimethyl-2,5-di(tert-butylperoxy)hexyne-3, di-tert-butyl peroxide, tert-butyl peroxyacetate, tert-butyl peroxy-2-ethylhexanoate, tert-amyl peroxy-2-ethylhexanoate, tert-butyl peroxydiethylacetate, tert-butyl peroxyisobutyrate, 1,1,3,3-tetramethylbutyl peroxy-2-ethylhexanoate,and mixtures thereof.

10. A polymer composition comprising:
at least one organic peroxide;
at least one polymer; and
at least one unsaturated scorch retarder having the structure of general formula (I): 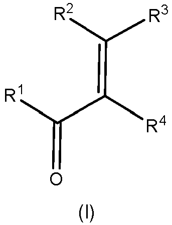 wherein,
R¹ is selected from OH, O-R⁵, or a saturated or unsaturated, linear, branched, or cyclic C₁ to C₈ alkyl group;
R² is on each occurrence independently selected from H or a saturated or unsaturated, linear or branched C₁ to C₈ alkyl group;
R³ is on each occurrence independently selected from H, O-R⁶, or a saturated or unsaturated, linear or branched C₁ to C₈ alkyl group;
R⁴ is on each occurrence independently selected from OH, O-R⁶, or a saturated or unsaturated, linear or branched C₁ to C₈ alkyl group;
R⁵ is selected from a saturated or unsaturated, linear or branched C₁ to C₈ alkyl group or -[CH₂]ₙ-OCOCR⁴CR²R³;
R⁶ is on each occurrence independently selected from a saturated or unsaturated, linear or branched C₁ to C₈ alkyl group; and
n is from 2-8,
with the proviso that one but not both of R² and R³ are H,
or wherein
R¹ and R² are joined to form a 5, 6, or 7 membered ring, said ring being formed of atoms selected from C, O, and S and containing one or two carbon-carbon double bonds and one or two carbonyl groups, said ring being optionally substituted with one or more saturated or unsaturated, linear or branched C₁ to C₈ alkyl group;
R³ is H; and
R⁴ and R⁶ are as described above,
or wherein
R³ and R⁴ are joined to form a 5, 6, or 7 membered ring, said ring being formed of atoms selected from C, O, and S and containing one or two carbon-carbon double bonds, said ring optionally substituted with one or more saturated or unsaturated, linear or branched C₁ to C₈ alkyl group;
R² is H; and
R¹, R⁵, R⁶ and n are as described above.

11. A polymer composition as claimed in claim 10, wherein the at least one polymer is selected from ethylene-vinylacetate (EVA), low density polyethylene (LDPE), high density polyethylene (HDPE), polyolefin elastomers (POE), nitrile rubber (NBR), hydrogenated nitrile rubber (HNBR), carboxylated nitrile butadiene rubber (XNBR), and mixtures thereof.

12. A polymer composition as claimed in claim 10 or claim 11, further comprising at least one crosslinking coagent, preferably selected from TAIC (triallyl isocyanurate), TAC (triallyl cyanurate), trimethylolpropane trimethacrylate, propoxylated 3-trimethylolpropane triacrylate, trimethylolpropane triacrylate, and mixtures thereof.

13. A polymer composition comprising:
at least one organic peroxide;
at least one polymer selected from ethylene-vinylacetate (EVA), low density polyethylene (LDPE), high density polyethylene (HDPE), polyolefin elastomers (POE), nitrile rubber (NBR), hydrogenated nitrile rubber (HNBR), carboxylated nitrile butadiene rubber (XNBR), and mixtures thereof; and
at least one unsaturated scorch retarder having the structure of general formula (II): 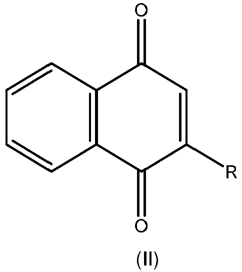 wherein
R is selected from OH, O-R", or a saturated or unsaturated, linear or branched C₁ to C₈ alkyl group; and
R" is a saturated or unsaturated, linear or branched C₁ to C₈ alkyl group.

14. A polymer composition comprising:
at least one organic peroxide selected from tert-butylperoxy 2-ethylhexyl carbonate, tert-amylperoxy 2-ethylhexyl carbonate, and mixtures thereof;
1,1'-(1,1-dimethyl-3-methylene-1,3-propanediyl)bisbenzene (a-MSD); and
at least one polymer selected from ethylene-vinylacetate (EVA), polyolefin elastomers (POE), and mixtures thereof.
